# EUROPÄISCHE PATENTSCHRIFT

(11) **EP 2 734 493 B1**
(45) Veröffentlichungstag und Bekanntmachung des Hinweises auf die Patenterteilung: **19.08.2015**
(21) Anmeldenummer: 12735152.6
(22) Anmeldetag: 16.07.2012
(51) Int. Cl.: C07C 29/128, C07C 31/38, C07C 67/10, C07C 69/63

(54) **VERFAHREN ZUR HERSTELLUNG 2,2-DIFLUORETHANOL**
PROCESS FOR PREPARING 2,2-DIFLUOROETHANOL
PROCÉDÉ DE PRÉPARATION DE 2,2-DIFLUOROÉTHANOL

(30) Priorität: 19.07.2011 EP 11174510; 20.07.2011 US 201161509800 P
(43) Veröffentlichungstag der Anmeldung: 28.05.2014
(73) Patentinhaber: Bayer Intellectual Property GmbH, 40789 Monheim (DE)
(72) Erfinder: MÜLLER, Thomas, Norbert, 40789 Monheim (DE); LUI, Norbert, 51519 Odenthal (DE); MOCZARSKI, Stefan, 51069 Köln (DE)
(74) Vertreter: BIP Patents
(86) Internationale Anmeldenummer: PCT/EP2012/063898
(87) Internationale Veröffentlichungsnummer: WO 2013/010985

(56) Entgegenhaltungen:
- WO-A1-2009/040367
- DATABASE WPI Week 198802 Thomson Scientific, London, GB; AN 1988-010746 XP002666811, & JP 62 273925 A (ASAHI CHEM IND CO LTD) 28. November 1987 (1987-11-28) in der Anmeldung erwähnt
- DAISUKE SHIBUTA ET AL: "Effect of solvent on the esterification between 1,1,1-trifluorochloroethane and akaline acetate", JOURNAL OF FLUORINE CHEMISTRY, Bd. 29, Nr. 1-2, 1. August 1985 (1985-08-01), Seite 176, XP055016072, ISSN: 0022-1139, DOI: 10.1016/S0022-1139(00)83412-0

## Beschreibung

Die vorliegende Erfindung betrifft ein Verfahren zur Herstellung von 2,2-Difluorethanol ausgehend von 2,2-Difluor-1-chlorethan(1-Chlor-2,2-difluorethan).

2,2-Difluorethanol ist eine wichtige Zwischenstufe bei der Synthese agrochemischer und pharmazeutischer Wirkstoffe. Es sind verschiedene Verfahren zur Herstellung fluorierter Alkohole bekannt. Viele der Verfahren verlaufen über katalytische Hydrierung bzw. durch Einsatz von Reduktionsmitteln.

Henne et al. beschreiben zum Beispiel in J. Am. Chem. Soc. 1952, 74, 1426-1428 die Reduktion eines *in situ* gebildeten Difluoressigsäurechlorids durch Lithiumaluminiumhydrid, wobei Difluorethanol in 69% Ausbeute erzeugt wird. Wirtschaftlich nachteilig ist der stöchiometrische Einsatz teurer Hydridquellen.

Booth et al. beschreiben in Tetrahedron 1990, 46, 2097-2110 die Reduktion von Difluoressigsäure mit Boran-Dimethylsulfid-Komplex wobei Difluorethanol in 55 % Ausbeute erhalten wird.

EP-1 820 789 A1 beschreibt die Reduktion von fluorierten Carbonsäuren, Carbonsäurehalogeniden, oder Carbonsäureestern mit Wasserstoff in Gegenwart eines Katalysators. Die dort beschriebene Methode soll sich insbesondere zur Herstellung von Difluorethanol (CF₂HCH₂OH) eignen, wobei vorzugsweise von fluorierten Estern ausgegangen wird, insbesondere von Difluoressigsäuremethylester bzw. - ethylester. Die Reaktion findet bei erhöhtem Druck statt, und als Katalysator wird Iridium, Rhodium, oder Ruthenium auf Kohle verwendet. Die Druckschrift beschreibt, dass ausgehend von Difluoressigsäuremethylester durch katalytische Hydrierung unter Verwendung eines Rh/C Katalysators bei 40 bar nach 18 Stunden das gewünschte Difluorethanol in einer Ausbeute von 74,4 % erhalten wurde. Nachteilig bei diesem Verfahren ist zum Einen das Verwenden eines teuren Edelmetallkatalysators und zum Anderen, dass die Reaktion bei hohem Druck ausgeführt wird, was zur Folge hat, dass die Reaktion in speziellen Hochdruckapparaturen durchgeführt werden muss.

WO 2007/071841, die sich mit der Herstellung von Difluorethanol beschäftigt, verwendet als Ausgangsmaterial für eine (dreifache) katalytische Hydrierung eine Verbindung CF₂XC(O)X, wobei Hal für Cl, Br, oder Jod steht (insbesondere Chlordifluoressigsäurechlorid). Als Katalysatoren werden insbesondere Ruthenium, Rhodium, Palladium, Osmium, Iridium, und Platin, die auf einem Träger aufgebracht sind, verwendet. Der Träger sollte dabei gleichfalls die Funktion einer Lewis-Säure besitzen und insbesondere Aluminium-Ionen beinhalten (z.B. Zeolithe oder Montmorillonit). Die Reaktion kann in der Gasphase, dann vorzugsweise bei einer Temperatur von 200 bis 300 °C und einem Wasserstoffdruck von vorzugsweise 1 bis 5 bar stattfinden. Gleichfalls kann die Reaktion in der flüssigen Phase stattfinden, dann liegt die Reaktionstemperatur zwischen 40 und 70 °C. Der Wasserstoffdruck ist vorzugsweise zwischen 10 und 20 bar. Die Reaktion in der Gasphase wird als vorteilhaft herausgestellt, denn sie lieferte bessere Ausbeuten an Difluorethanol und eine höhere Konversionsrate.

WO 2009/040367 beschreibt ein Verfahren zur Herstellung von 2,2-Difluorethanol. Hierzu wird in einer ersten Stufe das 1-Brom-2,2-difluorethan ausgehend von Difluorvinyliden hergestellt. In einer zweiten Stufe wird die Verbindung mit einem Sauerstoff-Nucleophil, wie z.B. Natrium- oder Kaliumsalze von Essig- oder Ameisensäure umgesetzt. WO 2009/040367 beschreibt ferner, dass das Bromatom im 1-Brom-2,2-difluorethan durch Reaktion mit Magnesium, Zink, Lithium oder Kupfer (insbesondere NaI oder KI) vor Reaktion mit dem Sauerstoff-Nucleophil aktiviert wird.

Im speziellen beschreibt WO2009/040367 die Herstellung von Difluorethanol indem in Stufe 2 Difluorbromethan mit Natriumacetat (= Natriumsalz der Essigsäure) in Gegenwart von Kaliumiodid, durch Erhitzen auf 130 °C in DMF für 18 h, umgesetzt wird, gefolgt von einer basenkatalysierten Umesterung in Gegenwart von Methanol. Das dabei gebildete Difluorethylacetat kann zunächst in einem Zwischenschritt durch Destillation isoliert werden oder direkt zum Difluorethanol umgesetzt werden. Ausgehend von eingesetztem Difluorbromethan liegen die Ausbeuten zwischen 56,8 und 87 %. Das hier beschriebene Verfahren ist aufwendig und relativ teuer und verlangt viele Zwischenschritte um zum gewünschten Difluorethanol zu gelangen. Will man nur Schritt 2 durchführen, muss man das teure Difluorbromethan kaufen.

Die japanische Veröffentlichung JP 62-273925A (=JP 1987-273925A) beschreibt die Herstellung von 2,2-Difluorethanol ausgehend von 1-Chlor-2,2-difluorethan mit Butyrolacton in Gegenwart von Wasser und Kaliumhydroxid. Die Reaktionsmischung wird hierfür auf 200 °C im Autoklaven für 2,5 h erhitzt, wobei 2,2-Difluorethanol in nur 48,6 %-iger Ausbeute bei 86 % Umsatz des Difluorchlorethans erhalten wird.

All die vorgenannten Verfahren zum Herstellen von 2,2-Difluorethanol sind nicht optimal. Viele der Verfahren verwenden teure Katalysatoren und man muss unter Druck arbeiten, was großtechnisch immer mit einem hohen Aufwand verbunden ist. Andere Verfahren (z.B. das aus WO 2009/040367) bestehen aus mehreren Verfahrensschritten, laufen über das teure 1-Brom-difluorethan, das zur besseren Reaktion auch noch aktiviert werden muss, oder verwenden das billigere 1-Chlor-2,2-difluorethan, wobei die erzielte Ausbeute und Selektivität von 48,6 % bei 86 % Umsatz des Difluorchlorethans nur sehr gering ist, was auf das Verwenden des unreaktiven 1-Chlor-2,2-difluorethans zurückzuführen ist.

Ausgehend von den bekannten Verfahren stellte sich nun die Aufgabe, ein Verfahren zur Herstellung von 2,2-Difluorethanol bereitzustellen, das einfach und kostengünstig ist, das als Ausgangsverbindung eine Verbindung verwendet, die zu einem verhältnismäßig günstigen Preis käuflich erhältlich ist und mit dem 2,2-Difluorethanol in hoher Ausbeute und guter Reinheit erhalten wird. Gleichfalls ist es wünschenswert, ein Verfahren bereitzustellen, das eine geringe Anzahl an Reaktionsschritten benötigt und das weitgehend ohne Reaktionshilfsmittel auskommt und, wenn möglich, das nicht in einem Druckbehälter durchgeführt werden muss.

Die Erfinder haben nun überraschenderweise gefunden, dass 1-Chlor-2,2-difluorethan einfach in einer nucleophilen Substitutionsreaktion zu einem Carbonsäuredifluorethylester umgesetzt werden kann, der dann durch basenkatalysierte Umesterung in Gegenwart eines Alkohols zum 2,2-Difluorethan weiter reagiert.

Überraschend insofern, als dass allgemein bekannt ist, dass Alkylchloride in nucleophilen Substitutionsreaktionen eine deutlich geringere Reaktivität aufweisen als die entsprechenden Alkylbromide und -iodide (March, Advanced Organic Chemistry 5th Edition, Kapitel 10, John Wiley & Sons, New York 2001). Die geringen Ausbeuten aus JP 62-273925 belegen dies.

Überraschend auch insofern, als dass das erfindungsgemäße Verfahren in gewöhnlichen Reaktionsgefäßen ausgeführt werden kann, obwohl 1-Chlor-2,2-difluorethan einen Siedepunkt von nur 35 °C besitzt und somit leicht flüchtig ist. Um bei leicht flüchtigen Substanzen eine ausreichend hohe Umsatzgeschwindigkeit zu erreichen muss die Reaktion bei erhöhten Temperaturen und unter Druck durchgeführt werden.

Die Anmeldung betrifft somit ein Verfahren zur Herstellung von 2,2-Difluorethanol umfassend die folgenden Schritte:
Schritt (i): Umsetzen von 1-Chlor-2,2-difluorethan mit einem Alkalimetallsalz der Ameisensäure oder Essigsäure in einem geeigneten Lösungsmittel zum entsprechenden 2,2-Difluorethylformiat oder 2,2-Difluorethylacetat, dadurch gekennzeichnet dass 1-Chlor-2,2-difluorethan in eine auf die gewünschte Reaktionstemperatur erwärmte Mischung aus Lösungsmittel und Alkalimetallsalz der Ameisensäure oder Essigsäure langsam zugegeben wird;
Schritt (ii): Umesterung des 2,2-Difluorethylformiats oder 2,2-Difluorethylacetats aus Schritt (i) in Gegenwart eines Alkohols (vorzugsweise Methanol) und gegebenenfalls einer Base.

Die Reaktion lässt sich folgendermaßen darstellen:

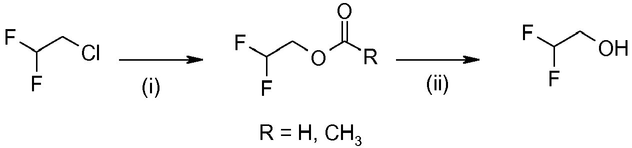

Das Verwenden von 1-Chlor-2,2-difluorethan hat den Vorteil, dass es günstiger als 1-Brom-2,2-difluorethan und zudem kommerziell in größeren Mengen erhältlich ist.

Durch das langsame Zugeben von 1-Chlor-2,2-difluorethan zu der erwärmten Mischung aus dem Alkalimetallsalz der Ameisensäure oder Essigsäure und Lösungsmittel wird eine vollständige und einfache Umsetzung zum gewünschten Produkt erreicht ohne dass unter erhöhtem Druck gearbeitet werden muss und ohne dass Reaktionshilfsmittel (z.B. Katalysatoren, Additive) eingesetzt werden müssen. Gleichfalls ist die Reaktionszeit vergleichsweise kurz. Das hat den Vorteil, dass die Reaktion einfach und kostengünstig durchgeführt werden kann und dass sie zudem umweltfreundlich ist, da sie keine Hilfschemikalien benötigt.

Erfindungsgemäß wird unter dem Ausdruck "langsames Zugeben" das portionsweise oder tropfenweise Zugeben von 1-Chlor-2,2-difluorethan über einen längeren Zeitraum verstanden. Die Länge des Zeitraums bemisst sich an der Größe des Reaktionsansatzes, und somit an der Menge des zuzugebenden 1-Chlor-2,2-difluorethans und lässt sich vom Fachmann durch Routinemethoden ermitteln. Entscheidend ist, dass das langsam zugegebene 1-Chlor-2,2-difluorethan genug Zeit hat, mit dem Alkalimetallsalz der Ameisensäure oder Essigsäure abzureagieren. Die Reaktionsdauer von Schritt (i) im erfindungsgemäßen Verfahren wird demnach so gewählt, dass eine vollständige Umsetzung von 1-Chlor-2,2-difluorethan gewährleistet ist. Die Reaktionsdauer kann im Bereich von 0,1 bis 12 h liegen. Bevorzugt wird das Reaktionssystem so eingestellt, dass die Reaktionsdauer im Bereich von 0,25 bis 5 h und besonders bevorzugt im Bereich von 0,5 bis 2 oder 3 h liegt.

In Schritt (i) des erfindungsgemäßen Verfahrens wird bevorzugt Natrium- oder Kaliumacetat, oder Natrium- oder Kaliumformiat eingesetzt, besonders bevorzugt wird Kaliumacetat oder Kaliumformiat verwendet.

Das in Schritt (i) eingesetzte Alkalimetallsalz der Ameisensäure oder Essigsäure wird im etwa 1- bis etwa 10-fachen molaren Überschuss, bevorzugt im etwa 1- bis etwa 2-fachen molaren Überschuss, und besonders bevorzugt im 1,1- bis 1,5-fachen molaren Überschuss, bezogen auf das eingesetzte 1-Chlor-2,2-difluorethan, verwendet.

Das in dem erfindungsgemäßen Verfahren verwendete Lösungsmittel wird vorzugsweise in einer solchen Menge eingesetzt, dass das Reaktionsgemisch während des ganzen Verfahrens gut rührbar bleibt. Vorteilhafterweise wird, bezogen auf das eingesetzte 2,2-Difluor-1-chlorethan, die 1- bis 50-fache Lösungsmittelmenge (v/v), bevorzugt die 2- bis 40-fache Lösungsmittelmenge (v/v), besonders bevorzugt die 2- bis 20-fache Lösungsmittelmenge (v/v) verwendet.

Erfindungsgemäße Lösungsmittel in Schritt (i) sind insbesondere organische Lösungsmittel (allein oder als Mischung mit anderen organischen Lösungsmitteln), die einen Siedepunkt über 70 °C haben und unter den Reaktionsbedingungen inert sind. Bevorzugte Lösungsmittel zur Verwendung in Schritt (i) sind Dimethylsulfoxid, Tetramethylensulfoxid, Dipropylsulfoxid, Benzylmethylsulfoxid, Diisobutylsulfoxid, Dibutylsulfoxid, Diisoamylsulfoxid; *N,N-*Dimethylacetamid, *N-*Methyl-formamid, *N,N-*Dimethyl-formamid, *N,N-*Dipropyl-formamid, *N,N-*Dibutyl-formamid, *N-*Methyl-pyrrolidon, *N-*Methyl-caprolactam und Mischungen davon, besonders bevorzugt sind N,N-Dimethylacetamid, *N-*Methylpyrrolidon, *N*,*N-*Dimethylformamid, Dimethylsulfoxid, Tetramethylensulfoxid und Mischungen davon, ganz besonders bevorzugt ist Dimethylsulfoxid oder *N-*Methylpyrrolidon und Mischungen davon.

Das langsame Zugeben in Schritt (i) des erfindungsgemäßen Verfahrens erfolgt bei der gewünschten Reaktionstemperatur, wobei unter Reaktionstemperatur die Innentemperatur verstanden wird. Die Reaktionstemperatur liegt gewöhnlich im Bereich von 70 °C bis 200 °C, bevorzugt im Bereich von 80 °C bis 160 °C, besonders bevorzugt im Bereich von 90°C bis 150 °C.

Das erfindungsgemäße Verfahren wird grundsätzlich unter Normaldruck durchgeführt. Es kann aber alternativ auch in einem druckstabilen geschlossenen Versuchsgefäß (Autoklav) durchgeführt werden. Der Druck während der Reaktion (d.h. der Eigendruck) ist dann abhängig von der verwendeten Reaktionstemperatur, dem verwendeten Lösungsmittel, und der Menge an verwendeten Edukten. Ist eine Druckerhöhung gewünscht, so kann eine zusätzliche Druckerhöhung mittels Zugabe eines Inertgases, wie Stickstoff oder Argon, durchgeführt werden.

Schritt (i) im erfindungsgemäßen Verfahren wird grundsätzlich in Abwesenheit eines Reaktionshilfsmittels (z.B. Katalysatoren oder Additive) durchgeführt. Es ist aus chemischer Sicht möglich, zur Aktivierung von 1-Chlor-2,2-difluorethan ein Reaktionshilfsmittel / Katalysator zur Mischung aus Alkalimetallsalz der Ameisensäure oder Essigsäure und Lösungsmittel zuzugeben. Denkbar ist der Einsatz von Alkalimetalliodiden und -bromiden (z.B. Natriumiodid, Kaliumiodid, Natriumbromid oder Kaliumbromid). Gleichfalls könnten quarternäre Ammoniumsalze der Form NR₄⁺X⁻, worin R für C₁₋₁₂-Alkyl und X für Br oder I stehen (z.B. Tetrabutylammoniumbromid, Tetrabutylammoniumiodid sowie Tricaprylmethylammoniumbromid) eingesetzt werden. Mögliche Konzentrationen der Katalysatoren liegen im Bereich von 0,001 bis 0,1 Äquivalente bezogen auf das eingesetzte 1-Chlor-2,2-difluorethan.

Die Umesterung in Schritt (ii) ist basenkatalysiert. Schritt (ii) kann mit der Reaktionsmischung aus Schritt (i), d.h. ohne Isolierung des in Schritt (i) hergestellten 2,2-Difluorethylformiats oder 2,2-Difluorethylacetats durchgeführt werden, wobei dann keine Base zum Reaktionsgemisch hinzugefügt werden muss, da sie bereits im Reaktionsgemisch vorhanden ist (z.B. Alkalimetallsalz der Ameisensäure oder Essigsäure aus Schritt (i)). Es ist bevorzugt im Schritt (ii) die Reaktionsmischung aus Schritt (i) ohne weiteren Isolierungsschritt zu verwenden.

Selbstverständlich kann im Schritt (ii) auch isoliertes 2,2-Difluorethylformiat oder 2,2-Difluorethylacetat verwendet werden. Hierfür kann die nach Schritt (i) erhaltene Reaktionsmischung aufgearbeitet und 2,2-Difluorethylformiat oder 2,2-Difluorethylacetat isoliert werden. Diese Ester können ferner destillativ abgetrennt werden. Wird das in Schritt (i) gewonnene 2,2-Difluorethylformiat oder 2,2-Difluorethylacetat nach Schritt (i) isoliert, muss in Schritt (ii) eine Base zugegeben werden.

Schritt (ii) erfolgt gewöhnlich in Substanz, d.h. ohne Zugabe von (weiteren) Lösungsmitteln, wobei der in Schritt (ii) verwendete Alkohol als Lösungsmittel dient.

Die Umesterung erfolgt indem man zu der Reaktionsmischung aus Schritt (i) oder zu dem isolierten Ester, gegebenenfalls die Base und den Alkohol zugibt. Vor allem bei Einsatz des isolierten Esters wird auf die Zugabe eines Lösungsmittels verzichtet. Die so erhaltene Mischung wird bei Raumtemperatur oder unter Rückflussbedingungen für 0,5 bis 2 h gerührt.

In Schritt (ii) verwendbare Alkohole, die gleichzeitig als Lösungsmittel dienen können, sind Methanol, Butanol, Isobutanol, Pentanol und seine Isomeren, Hexanol und seine Isomeren, bevorzugt wird Methanol verwendet. Der Alkohol wird im 1- bis 40-fachen Überschuss, bevorzugt im 1,5- bis 10-fachen Überschuss und besonders bevorzugt im 2- bis 5-fachen Überschuss eingesetzt.

Beispiele für die in Schritt (ii) benötigten erfindungsgemäßen Basen sind Alkalimetallhydroxide, und Alkalimetallmethanolat in fester Form oder als Lösung in Methanol, Alkalimetallcarbonate, Alkalimetallacetate, Alkalimetallformiate und Alkalimetallphosphate. Bevorzugte Basen sind Natriummethanolat, Natriumhydroxid und Kaliumacetat. Die Menge an zugesetzter Base beträgt 0,001 bis 0,1 Äquivalente bezogen auf das eingesetzte 2,2-Difluorethylformiat oder 2,2-Difluorethylacetat.

Die Aufarbeitung (Reinigung) des 2,2-Difluorethanols erfolgt durch Destillation.

Die vorliegende Erfindung wird anhand der nachfolgenden Beispiele näher erläutert, wobei die Beispiele nicht in die Erfindung einschränkender Weise zu interpretieren sind.

### Herstellungsbeispiele:

### Beispiel 1

### Schritt (i): Herstellung von 2,2-Difluorethylacetat

In einem Dreihalskolben mit mechanischem Rührer, Tropftrichter und Trockeneiskühler werden 148 g (1,475 mol) Kaliumacetat in 300 mL Dimethylsulfoxid vorgelegt und auf 120 °C erhitzt. Anschließend wird eine Mischung aus 100 g (0,983 mol) 2,2-Difluor-1-chlorethan in 100 mL Dimethylsulfoxid innerhalb einer Stunde zugetropft. Die Reaktionsmischung wird 1,5 h bei 120 °C nachgerührt und auf Raumtemperatur abgekühlt. Eine Reaktionskontrolle über Gaschromatographie zeigt vollständigen Umsatz des 2,2-Difluor-1-chlorethans. Nach Destillation wird das gewünschte 2,2-Difluorethylacetat in 90,8 %-iger Ausbeute erhalten.

NMR ¹H (CDCl₃): 5.94 (tt, 1H, *J* = 3.9 Hz, 55.1 Hz), 4.27 (dt, 2H, *J* = 4.0 Hz, 13.7 Hz), 2.14 (s, 3H) NMR ¹⁹F (CDCl₃): -126.24 (td, *J* = 13.7 Hz, 55.1 Hz)

### Schritt (ii): Herstellung von 2,2-Difluorethanol aus 2,2-Difluorethylacetat

In einem Dreihalskolben mit mechanischem Rührer und Rückflusskühler werden 112 g (889 mmol) 2,2-Difluorethylacetat mit 100 g (3.11 mol) Methanol versetzt und 2.14 g (53 mmol) festes Natriumhydroxid zugegeben. Die Reaktionsmischung wird 1 h bei Raumtemperatur gerührt. Eine Reaktionskontrolle über Gaschromatographie zeigt vollständigen Umsatz des Einsatzmaterials. Nach Destillation wird das Zielprodukt in 74,4 %iger Ausbeute erhalten.

NMR ¹H (CDCl₃): 5.85 (tt, 1H, *J* = 3.9 Hz, 55.8 Hz), 3.84 - 3.78 (m, 2H), 2.02 (br t, 1H, *J* = 6.7 Hz) NMR ¹⁹F (CDCl₃): -128.3 (td, *J* = 14.4 Hz, 55.8 Hz)

### Beispiel 2 - ohne Isolierung von 2,2-Difluorethylacetat

Schritt (i): In einem Dreihalskolben mit mechanischem Rührer, Tropftrichter und Trockeneiskühler werden 289,5 g (2,95 mol) Kaliumacetat in 600 mL Dimethylsulfoxid vorgelegt und auf 120 °C erhitzt und anschließend eine Mischung aus 200 g (1,97 mol) 2,2-Difluor-1-chlorethan in 200 mL Dimethylsulfoxid innerhalb von 30 Minuten zugetropft. Die Reaktionsmischung wird 2 h nachgerührt und auf 60 °C abgekühlt. Eine Reaktionskontrolle über Gaschromatographie zeigt vollständigen Umsatz des 2,2-Difluor-1-chlorethans.

### Schritt (ii): Es werden innerhalb von 20 Minuten 221 g (6,88 mol) Methanol zugetropft und die Reaktionsmischung anschließend für 2h auf 90 °C erwärmt.

Eine Reaktionskontrolle mittels Gaschromatographie zeigte vollständigen Umsatz des 2,2-Difluorethylacetats. Nach Destillation wird das 2,2-Difluorethanol in 84,4 %-iger Ausbeute erhalten.

NMR ¹H (CDCl₃): 5.85 (tt, 1H, *J* = 3.9 Hz, 55.8 Hz), 3.84 - 3.78 (m, 2H), 2.02 (br t, 1H, *J* = 6.7 Hz) NMR ¹⁹F (CDCl₃): -128.3 (td, J= 14.4 Hz, 55.8 Hz)

## Patentansprüche

1. Verfahren zur Herstellung von 2,2-Difluorethanol umfassend die folgenden Schritte:
Schritt (i): Umsetzen von 1-Chlor-2,2-difluorethan mit einem Alkalimetallsalz der Ameisensäure oder Essigsäure in einem geeigneten Lösungsmittel zum entsprechenden 2,2-Difluorethylformiat oder 2,2-Difluorethylacetat, **dadurch gekennzeichnet dass** 1-Chlor-2,2-difluorethan in eine auf die gewünschte Reaktionstemperatur erwärmte Mischung aus Lösungsmittel und Alkalimetallsalz der Ameisensäure oder Essigsäure langsam zugegeben wird;
Schritt (ii): Umesterung des 2,2-Difluorethylformiats oder 2,2-Difluorethylacetats aus Schritt (i) in Gegenwart eines Alkohols und gegebenenfalls einer Base.

2. Verfahren nach Anspruch 1, wobei das geeignete Lösungsmittel ausgewählt ist unter Dimethylsulfoxid, Tetramethylensulfoxid, Dipropylsulfoxid, Benzylmethylsulfoxid, Diisobutylsulfoxid, Dibutylsulfoxid, Diisoamylsulfoxid; *N*,*N*-Dimethylacetamid, *N-*Methylformamid, *N,N*-Dimethyl-formamid, *N*,*N-*Dipropyl-formamid, *N*,*N-*Dibutyl-formamid, *N-*Methyl-pyrrolidon, *N-*Methyl-caprolactam und Mischungen davon.

3. Verfahren nach Anspruch 1 oder 2, wobei die Reaktionstemperatur in Schritt (i), bei der das 1-Chlor-2,2-difluorethan zugegeben wird, im Bereich von 70 °C bis 200 °C liegt.

4. Verfahren nach einem der Ansprüche 1 bis 3, wobei unter langsamer Zugabe eine portionsweise oder tropfenweise Zugabe verstanden wird.

5. Verfahren nach einem der Ansprüche 1 bis 4, wobei das 2,2-Difluorethylformiat oder das 2,2-Difluorethylacetat nach Schritt (i) durch Destillation von der Reaktionsmischung abgetrennt wird und wobei in Schritt (ii) eine Base zugegeben wird.

6. Verfahren nach Anspruch 5, wobei die Base ausgewählt ist aus Alkalimetallhydroxiden, Alkalimetallmethanolaten, Alkalimetallcarbonaten, Alkalimetallacetaten, Alkalimetallformiaten und Alkalimetallphosphaten.

7. Verfahren nach einem der Ansprüche 1 bis 4, wobei die Reaktionsmischung aus Schritt (i) ohne weitere Aufreinigung oder einem Isolierungsschritt in Schritt (ii) eingesetzt wird, wobei dann in Schritt (ii) keine Base zugeben wird.

## Claims

1. Process for preparing 2,2-difluoroethanol, comprising the following steps:
step (i): reacting 1-chloro-2,2-difluoroethane with an alkali metal salt of formic acid or acetic acid in a suitable solvent to give the corresponding 2,2-difluoroethyl formate or 2,2-difluoroethyl acetate, **characterized in that** 1-chloro-2,2-difluoroethane is added gradually to a mixture, heated to the desired reaction temperature, of solvent and alkali metal salt of formic acid or acetic acid;
step (ii): transesterifying the 2,2-difluoroethyl formate or 2,2-difluoroethyl acetate from step (i) in the presence of an alcohol and optionally of a base.

2. Process according to Claim 1, wherein the suitable solvent is selected from dimethyl sulphoxide, tetramethylene sulphoxide, dipropyl sulphoxide, benzyl methyl sulphoxide, diisobutyl sulphoxide, dibutyl sulphoxide, diisoamyl sulphoxide; *N,N-*dimethylacetamide, N-methylformamide, *N,N-*dimethylformamide, *N*,*N*-dipropylformamide, *N,N-*dibutylformamide, N-methylpyrrolidone, N-methylcaprolactam and mixtures thereof.

3. Process according to Claim 1 or 2, wherein the reaction temperature in step (i) in which the 1-chloro-2,2-difluoroethane is added is in the range from 70°C to 200°C.

4. Process according to any of Claims 1 to 3, wherein gradual addition is understood to mean addition in portions or dropwise.

5. Process according to any of Claims 1 to 4, wherein the 2,2-difluoroethyl formate or the 2,2-difluoroethyl acetate is separated from the reaction mixture by distillation after step (i), and wherein a base is added in step (ii).

6. Process according to Claim 5, wherein the base is selected from alkali metal hydroxides, alkali metal methoxides, alkali metal carbonates, alkali metal acetates, alkali metal formates and alkali metal phosphates.

7. Process according to any of Claims 1 to 4, wherein the reaction mixture from step (i) is used in step (ii) without further purification or an isolation step, in which case no base is added in step (ii).

## Revendications

1. Procédé de fabrication de 2,2-difluoroéthanol comprenant les étapes suivantes :
étape (i) : la mise en réaction de 1-chloro-2,2-difluoroéthane avec un sel de métal alcalin de l'acide formique ou de l'acide acétique dans un solvant approprié pour former le formiate de 2,2-difluoroéthyle ou l'acétate de 2,2-difluoroéthyle correspondant, **caractérisé en ce que** le 1-chloro-2,2-difluoroéthane est ajouté lentement dans un mélange d'un solvant et d'un sel de métal alcalin de l'acide formique ou de l'acide acétique porté à la température de réaction souhaitée ;
étape (ii) : la transestérification du formiate de 2,2-difluoroéthyle ou de l'acétate de 2,2-difluoroéthyle de l'étape (i) en présence d'un alcool et éventuellement d'une base.

2. Procédé selon la revendication 1, dans lequel le solvant approprié est choisi parmi le diméthylsulfoxyde, le tétraméthylènesulfoxyde, le dipropylsulfoxyde, le benzylméthylsulfoxyde, le diisobutylsulfoxyde, le dibutylsulfoxyde, le diisoamylsulfoxyde, le *N,N-*diméthylacétamide, le *N-*méthylformamide, le *N,N-*diméthylformamide, le *N,N-*dipropylformamide, le *N,N*-dibutylformamide, la *N-*méthylpyrrolidone, le *N*-méthylcaprolactame et leurs mélanges.

3. Procédé selon la revendication 1 ou 2, dans lequel la température de réaction à l'étape (i), à laquelle le 1-chloro-2,2-difluoroéthane est ajouté, se situe dans la plage allant de 70 °C à 200 °C.

4. Procédé selon l'une quelconque des revendications 1 à 3, dans lequel un ajout lent se rapporte à un ajout en portions ou goutte à goutte.

5. Procédé selon l'une quelconque des revendications 1 à 4, dans lequel le formiate de 2,2-difluoroéthyle ou l'acétate de 2,2-difluoroéthyle est séparé du mélange réactionnel par distillation après l'étape (i) et une base est ajoutée à l'étape (ii).

6. Procédé selon la revendication 5, dans lequel la base est choisie parmi les hydroxydes de métaux alcalins, les méthanolates de métaux alcalins, les carbonates de métaux alcalins, les acétates de métaux alcalins, les formiates de métaux alcalins et les phosphates de métaux alcalins.

7. Procédé selon l'une quelconque des revendications 1 à 4, dans lequel le mélange réactionnel de l'étape (i) est utilisé dans l'étape (ii) sans purification supplémentaire ou étape d'isolement, aucune base n'étant alors ajoutée à l'étape (ii).
